# EUROPÄISCHE PATENTANMELDUNG

(11) **EP 1 161 937 A2**
(43) Veröffentlichungstag der Anmeldung: **12.12.2001**
(21) Anmeldenummer: 01113165.3
(22) Anmeldetag: 30.05.2001
(51) Int. Cl.: A61K 7/48, A61P 17/00

(54) **Kosmetische Stifte**

(30) Priorität: 09.06.2000 DE 10028718
(71) Anmelder: Henkel Kommanditgesellschaft auf Aktien, 40589 Düsseldorf (DE)
(72) Erfinder: Banowski, Bernhard, 40597 Düsseldorf (DE); Hengstermann, Dieter, 46325 Borken (DE); Heide, Barbara, 47809 Krefeld (DE); Frisoli, Christian, 41564 Kaarst (DE); Wadle, Armin, Dr., 40699 Erkrath (DE)

(57) **Zusammenfassung**

Gegenstand der Erfindung sind im wesentlichen wasserfreie geformte Zubereitungen, bevorzugt in der Form eines Stiftes, zur Applikation kosmetischer Wirk- und Pflegestoffe auf die Haut. Die Zubereitungen enthalten (A) ein- oder mehrwertige C₂₋₄-Alkohole, (B) im Bereich von 28 ― 130 °C schmelzende Fettkomponenten, (C) Siliconöle mit einem Siedepunkt unterhalb von 250 °C, (D) oberflächenaktive Komponenten und (E) teilchenförmige Komponenten. In einer bevorzugten Ausführungsform sind weiterhin bis zu 15 Gew.-% kosmetischer oder dermatologischer Wirkstoffe, bezogen auf die Gesamtzusammensetzung, enthalten. Die Stifte eignen sich bevorzugt zur kosmetischen Behandlung und Pflege von Problemen der Gesichts- und der Körperhaut, insbesondere überschüssige Sebumproduktion und Hautunreinheiten.

## Beschreibung

Die Erfindung betrifft im wesentlichen wasserfreie geformte Zubereitungen, bevorzugt in der Form eines Stiftes, zur Applikation kosmetischer Wirk- und Pflegestoffe auf die Haut.

Kosmetikstifte sind sogenannte Ein-Hand-Produkte. Man unterscheidet Stifte mit indirekter Applikation, bei denen die Stiftzusammensetzung mit einem Applikator, z. B. einem Haar- oder Schwammpinsel oder einer kleinen Bürste auf die Haut oder Schleimhaut aufgetragen wird, und die direkte Applikation, bei der die Stiftmine direkt über die Haut oder Schleimhaut geführt wird.

Bei den Verbrauchern erfreuen sich Stiftpräparate zur Anwendung sowohl dekorativer als auch pflegender Kosmetik hoher Beliebtheit. Sie sind handlich, transportstabil und bequem aufzutragen. Zunehmend besteht bei den Konsumenten das Bedürfnis nach Zusammensetzungen, die neben der Basisfunktion, also z. B. dem Abdecken von Hautunreinheiten oder dem Mattieren fettglänzender Haut, noch weitere Pflege- und Behandlungseffekte, z. B. durch antibakterielle oder hautberuhigende Wirkstoffe, und gleichzeitig ein angenehmes, frisches Hautgefühl vermitteln.

Aufgabe der Erfindung war die Entwicklung eines kosmetischen Stiftpräparates zur Pflege und Behandlung der Gesichts- und der Körperhaut. Die kosmetische Behandlung jugendlich-unreiner, fettiger Haut stand bei der Entwicklung der Stiftgrundlage im Vordergrund. Die direkte Applikation ist erfindungsgemäß bevorzugt. Daher muss die Stiftmasse sowohl die mechanische und thermische Stabilität des Präparates als auch einen ausreichenden Abrieb auf der Haut gewährleisten. Zur Behandlung von Hautunreinheiten und zur Verbesserung des Hautbildes sollen mit dem Stift zusätzliche kosmetische Wirkstoffe aufgetragen werden. Diese müssen in der Stiftmasse homogen verteilbar sein. Weiterhin soll die Anwendung des Stiftes ein angenehmes, erfrischendes Hautgefühl hinterlassen, um die besonderen Anforderungen der Konsumenten zufriedenzustellen.

Überraschenderweise wurde gefunden, dass man mit bestimmten Mischungen aus ein- oder mehrwertigen C₂₋₄-Alkoholen, im Bereich von 28-130°C schmelzenden Fettkomponenten, Siliconölen mit einem Siedepunkt unter 250°C, oberflächenaktiven Komponenten und teilchenförmigen Komponenten Stiftpräparate erhält, die den gestellten Anforderungen in hohem Maße genügen. Ein hoher Gehalt an teilchenförmigen Substanzen wie beispielsweise Talkum oder Stärke dient zur Sebumabsorption und zur Mattierung von unerwünschtem Hautglanz. Zu diesem Zweck muss die Stiftbasis im wesentlichen wasserfrei sein, d. h., der Wassergehalt beträgt, bezogen auf die Gesamtzusammensetzung, maximal 5 Gew.-%. Die Siliconöle mit einem Siedepunkt unter 250 °C erzeugen zusammen mit der Alkoholkomponente nach dem Auftragen durch ihre Verdunstung das gewünschte erfrischend-kühle Hautgefühl.

Die Druckschrift WO 96/27364 (Eastman) offenbart wasserfreie Stifte als Vehikel zum Auftragen kosmetischer Wirkstoffe. Die Zusammensetzungen enthalten keine sebumabsorbierenden Partikel und sind aufgrund ihres hohen Fett- und Ölgehaltes nicht zur Pflege fettiger Haut geeignet.

Die Druckschrift US 5225186 (Revlon) offenbart wasserfreie Stifte mit einem Gehalt an partikelförmigen Substanzen von 10 bis 80 Gew.-%. Der Stift eignet sich ebenfalls nicht zur Behandlung fettglänzender, unreiner Haut.

Die Patentschrift US 5972318 (L'Oréal) offenbart wasserfreie Stifte auf der Basis spezieller Siliconwachse und spezieller flüchtiger Siliconöle, die gegebenenfalls partikelförmige Substanzen enthalten können.

Die Druckschrift EP 602905 A2 (Revlon) offenbart Make-up-Zusammensetzungen, die 1 - 70 Gew.-% flüchtige Siliconöle und/oder C₈₋₂₀-Isoparaffine, 0,1 - 15 Gew.-% Siliconharze, 10 - 45 Gew.-% Wachse, 5 - 50 Gew.-% Puder einschließlich Pigmenten und 1 - 30 Gew.-% kosmetische Öle enthalten und beispielsweise in Stiftform vorliegen können.

Ein erster Gegenstand der Erfindung ist eine bei 40°C formstabile, auf der Haut verstreichbare kosmetische Stiftmasse, die dadurch gekennzeichnet ist, dass sie (A) ein- oder mehrwertige C₂₋₄-Alkohole, (B) im Bereich von 28-130°C schmelzende Fettkomponenten, (C) Siliconöle mit einem Siedepunkt unterhalb von 250 °C, (D) oberflächenaktive Komponenten und (E) teilchenförmige Komponenten enthält. Erfindungsgemäß schließen die Begriffe "Fettkomponente" und "Fettstoff" dabei nicht nur Fette im engeren Sinne, d. h. Triester von Fettsäuren mit Glycerin, sondern auchFettsäuremono- und diglyceride, Wachsester, d. h. die Ester langkettiger Fettsäuren mit langkettigen Fettalkoholen, und Paraffine ein.

Die ein- oder mehrwertigen C₂₋₄-Alkohole werden erfindungsgemäß ausgewählt aus Ethanol, 1-Propanol, 2-Propanol, iso-Propanol, 1-Butanol, 2-Butanol, Ethylenglykol, 1,2-Propylenglykol, 1,3-Butylenglykol und Glycerin.

In einer besonders bevorzugten Ausführungsform enthält die erfindungsgemäße Stiftmasse als Alkoholkomponente (A) Ethanol. Der Alkoholgehalt beträgt bevorzugt 10 - 30 Gew.-%, bezogen auf die Gesamtzusammensetzung.

In einer weiteren bevorzugten Ausführungsform umfasst die Fettkomponente (B) mindestens einen unterhalb von 50 °C schmelzenden Fettstoff und mindestens einen im Bereich von 50 °C und höher schmelzenden Fettstoff.

Besonders bevorzugt sind Stiftmassen, in denen das Mengenverhältnis der unter 50 °C schmelzenden Fettstoffe zu den ab 50°C schmelzenden Fettstoffen 1,5:1 bis 4:1 beträgt.
Der Anteil der Fettkomponente (B) an der Gesamtzusammensetzung beträgt bevorzugterweise 10 - 40 Gew.-%.

Die unterhalb von 50 °C schmelzenden Fettstoffe werden erfindungsgemäß ausgewählt aus den Mono-, Di- und Triglyceriden der C₁₂₋₁₄-Fettsäuren (z. B. Novata® AB), Estern von gegebenenfalls hydroxylierten C₂₋₄-Carbonsäuren mit Lanolinalkoholen und C₁₂₋₁₈-Fettalkoholen, acetyliertem Lanolin, Estern von gesättigten und ungesättigten C₁₂₋₁₈-Fettsäuren mit gesättigten und ungesättigten C₁₂₋₁₈-Fettalkoholen, Estern von C₂₋₁₀-Fettsäuren mit C₁₄-Fettalkohol, C₁₄-Fettsäureestern, Cholesterol- oder Lanosterolestern von C₁₀₋₃₀-Fettsäuren und ethoxylierten C₁₂₋₂₀-Fettsäureglykolestern. Bevorzugt werden die Mono-, Di- und Triglyceride der C₁₂₋₁₄-Fettsäuren, acetyliertes Lanolin, Isopropyllanolat, Myristylmyristat, Stearinsäureoleylester, Isopropylmyristat und Myristyllactat, besonders bevorzugt sind die Mono-, Di- und Triglyceride der C₁₂₋₁₄-Fettsäuren.

Die festigkeitsgebenden, ab 50 °C schmelzenden Fette und Wachse werden erfindungsgemäß ausgewählt aus Bienenwachs und anderen Insektenwachsen wie Chinawachs und Hummelwachs, Pflanzenwachsen wie Candelillawachs, Carnaubawachs, Japanwachs, Espartograswachs, Korkwachs, Reiswachs, Zuckerrohrwachs, Fruchtwachsen, Apfelwachs, Blütenwachsen, Blattwachsen von Nadelhölzern, Kaffeewachs, Flachswachs und Sesamwachs, Ozokerit, Mikrowachs, Ceresin, Paraffin, Triglyceriden gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, synthetischen Vollestern aus Fettsäuren und Glykolen (z. B. Syncrowachs® ) oder Polyolen mit 2 - 6 C-Atomen, Fettsäuremonoalkanolamiden mit einem C₁₂₋₂₂-Acylrest und einem C₂₋₄-Alkanolrest, synthetischen Fettsäure-Fettalkoholestern, z. B. Stearylstearat oder Cetylpalmitat, Esterwachsen aus natürlichen Fettsäuren und synthetischen C₂₀₋₄₀-Fettalkoholen (INCI-Bezeichnung C20-40 Alkyl Stearate, erhältlich als synthetisches Bienenwachs z. B. von der Firma Witco oder als Kesterwachs® von der Firma Koster Keunen Holland bv), und Vollestern aus Fettalkoholen und Di- und Tricarbonsäuren, z. B. Dicetylsuccinat oder Dicetyl-/ stearyladipat, sowie Mischungen dieser Substanzen. Bevorzugt sind die Triglyceride gesättigter und gegebenenfalls hydroxylierter C₁₆₋₃₀-Fettsäuren, vor allem hydriertes Rizinusöl, Glyceryltribehenat und Glyceryltri-12-hydroxystearat, außerdem Bienenwachs, synthetisches Bienenwachs, Candelillawachs, Carnaubawachs, Ozokerit, Mikrowachs, Ceresin und Paraffin. Besonders bevorzugt sind hydriertes Rizinusöl, Bienenwachs, synthetisches Bienenwachs, Ozokerit und Paraffin.

Die erfindungsgemäß enthaltenen Siliconöle werden ausgewählt aus Octamethylcyclotetrasiloxan, Decamethylcyclopentasiloxan, Dodecamethylcyclohexasiloxan und Dimethylpolysiloxan. Besonders bevorzugt sind Decamethylcyclopentasiloxan, Dodecamethylcyclohexasiloxan und Dimethylpolysiloxan.
Der Anteil der Siliconöle mit einem Siedepunkt unter 250 °C an der Gesamtzusammensetzung beträgt bevorzugterweise 10 - 40 Gew.-%.

Der Anteil der oberflächenaktiven Komponenten (D) an der Gesamtzusammensetzung beträgt bevorzugterweise 1 - 10 Gew.-%.
Die erfindungsgemäß eingesetzten oberflächenaktiven Substanzen ermöglichen als Emulgatoren das Einmischen der gegebenenfalls hydrophilen partikelförmigen Komponenten und der Wirkstoffe, die in wässriger Lösung vorliegen. Weiterhin erleichtern die Emulgatoren das Abwaschen der Zusammensetzung von der Haut. Erfindungsgemäß können alle Typen verwendet werden, also sowohl ionische (anionische, kationische, ampholytische, zwitterionische) als auch nichtionische oberflächenaktive Substanzen. Bevorzugt sind nichtionische und anionische oberflächenaktive Substanzen sowie Mischungen hiervon.

Nichtionische Surfactants enthalten als hydrophile Gruppe z. B. eine Polyolgruppe, eine Polyalkylenglykolethergruppe oder eine Kombination aus Polyol- und Polyglykolethergruppen. Solche Verbindungen sind beispielsweise:
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- mit einem Methyl- oder C₂-₆-Alkylrest endgruppenverschlossene Anlagerungsprodukte von 2 bis 50 Mol Ethylenoxid und/oder 0 bis 5 Mol Propylenoxid an lineare und verzweigte C₈₋₃₀-Fettalkohole, an C₈₋₃₀-Fettsäuren und an C₈₋₁₅-Alkylphenole, z. B. die von der Firma Cognis erhältlichen Handelsprodukte Dehydol® LS und Dehydol® LT,
- C₁₂-C₃₀-Fettsäuremono- und -diester von Anlagerungsprodukten von 1 bis 30 Mol Ethylenoxid an Glycerin,
- Anlagerungsprodukte von 5 bis 60 Mol Ethylenoxid an Rizinusöl und gehärtetes Rizinusöl,
- Fettsäureester von Polyolen wie Glycerin und Trimethylolpropan und deren Ethylenoxid-Anlagerungsprodukte, z. B. das Handelsprodukt Hydagen® HSP (Cognis) oder die Sovermol® - Typen (Cognis),
- alkoxylierte Triglyceride,
- alkoxylierte Fettsäurealkylester der Formel R¹CO-(OCH₂CHR²)ₙOR³, in der R¹CO für einen linearen oder verzweigten, gesättigten und/oder ungesättigten C₆₋₂₂-Acylrest, R² für Wasserstoff oder eine Methylgruppe, R³ für lineare oder verzweigte C₁₋₄-Alkylreste und n für Zahlen von 1 bis 20 steht,
- Aminoxide,
- Hydroxymischether, wie sie beipielsweise in der DE-OS 19738866 beschrieben sind,
- Sorbitanfettsäureester und Anlagerungsprodukte von Ethylenoxid an Sorbitanfettsäureester wie beispielsweise die Polysorbate,
- Zuckerfettsäureester und Anlagerungsprodukte von Ethylenoxid und Polyglycerin an Zuckerfettsäureester,
- Anlagerungsprodukte von Ethylenoxid und Polyglycerin an Fettsäurealkanolamide, Fettamine und Fettsäure-N-alkylglucamide,
- Alkylpolyglykoside entsprechend der allgemeinen Formel RO-(Z)ₓ wobei R für Alkyl, Z für Zucker sowie x für die Anzahl der Zuckereinheiten steht. Die erfindungsgemäß verwendbaren Alkylpolyglykoside können lediglich einen bestimmten Alkylrest R enthalten. Üblicherweise werden diese Verbindungen aber ausgehend von natürlichen Fetten und Ölen hergestellt. In diesem Fall liegen als Alkylreste R Mischungen entsprechend den Ausgangsverbindungen bzw. entsprechend der jeweiligen Aufarbeitung dieser Verbindungen vor. Besonders bevorzugt sind Alkylpolyglykoside, bei denen
   - R: - im wesentlichen aus C₈- und C₁₀-Alkylgruppen,
   - im wesentlichen aus C₁₂- und C₁₄-Alkylgruppen,
   - im wesentlichen aus C₈- bis C₁₆-Alkylgruppen oder
   - im wesentlichen aus C₁₂- bis C₁₆-Alkylgruppen oder
   - im wesentlichen aus C₁₆₋ bis C₁₈-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt, beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten durchschnittlich 1,1 bis 5 Zuckereinheiten. Alkylpolyglykoside mit x-Werten von 1,1 bis 2,0 sind bevorzugt. Besonders bevorzugt sind Alkylglykoside, bei denen x 1,1 bis 1,8 beträgt.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten;
- Gemische aus Alkyl-(oligo)-glucosiden und Fettalkoholen, zum Beispiel als Handelsprodukte Montanov® 68 oder Emulgade® PL 68/50 erhältlich,
- Wollwachsalkohole,
- Sterine, also Steroide, die am C-Atom 3 des Steroid-Gerüstes eine Hydroxylgruppe tragen und sowohl aus tierischem Gewebe (Zoosterine, z. B. Cholesterin, Lanosterin) wie auch aus pflanzlichen Fetten (Phytosterine, z. B. Ergosterin, Stigmasterin, Sitosterin) und aus Pilzen und Hefen (Mykosterine) isoliert werden;
- Phospholipide, die z. B. als Lecithine bzw. Phosphatidylcholine aus z. B. Eidotter oder Pflanzensamen (z. B. Sojabohnen) gewonnen werden;
- Polyglycerine und Polyglycerinderivate, beispielsweise Polyglycerinpoly-12-hydroxystearat (Handelsprodukt Dehymuls® PGPH der Firma Cognis).

Bevorzugte nichtionische oberflächenaktive Substanzen sind C₁₂-C₃₀-Fettsäuremono- und -diester von Anlagerungsprodukten von 1 bis 30 Mol Ethylenoxid an Glycerin, besonders bevorzugt PEG-20 Glycerylmonostearat (z. B. Cutina® E 24 PF), weiterhin Polyglycerine und Polyglycerinderivate, besonders bevorzugt Polyglycerinpoly-12-hydroxystearat (Dehymuls® PGPH), Polyglycerinpoly-3-diisostearat (Lameform® TGI) und Polyglycerinpolyricinoleat (Polymuls® PGPR), Alkyl-(oligo)-glucoside, Gemische aus Alkyl-(oligo)-glucosiden und Fettalkoholen, Partialester von Zuckem, Zuckerderivaten und Zuckeralkoholen mit gesättigten C₈₋₂₂-Fettsäuren, z. B. Sorbitfettsäureester, die als Handelsprodukte Arlacel® oder Span® von der Firma Uniqema erhältlich sind.

Als anionische Surfactants können in den erfindungsgemäßen Zubereitungen alle für die Verwendung am menschlichen Körper geeigneten anionischen oberflächenaktiven Stoffe eingesetzt werden. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 8 bis 30 Kohlenstoffatomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 bis 4 Kohlenstoffatomen in der Alkanolgruppe:
- lineare und verzweigte Fettsäuren mit 8 bis 30 C-Atomen (Seifen),
- Ethercarbonsäuren der Formel R⁴-O-(CH₂-CH₂O)ₓ-CH₂-COOH, in der R⁴ eine lineare Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- lineare Alkansulfonate mit 8 bis 24 C-Atomen,
- lineare Alpha-Olefinsulfonate mit 8 bis 24 C-Atomen,
- Alpha-Sulfofettsäuremethylester von Fettsäuren mit 8 bis 30 C-Atomen,
- Alkylsulfate und Alkylpolyglykolethersulfate der Formel R⁵-O(CH₂-CH₂O)ₓ-OSO₃H, in der R⁵ eine bevorzugt lineare Alkylgruppe mit 8 bis 30 C-Atomen und x = 0 oder 1 bis 12 ist,
- gemischte oberflächenaktive Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 8 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an C₈₋₂₂-Fettalkohole darstellen,
- Alkyl- und/oder Alkenyletherphosphate der Formel (I), 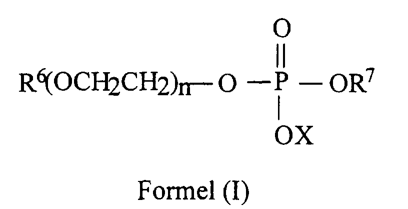 in der R⁶ bevorzugt für einen aliphatischen Kohlenwasserstoffrest mit 8 bis 30 Kohlenstoffatomen, R⁷ für Wasserstoff, einen Rest (CH₂CH₂O)ₙR⁶ oder X, n für Zahlen von 1 bis 10 und X für Wasserstoff, ein Alkali- oder Erdalkalimetall oder NR⁸R⁹R¹⁰R¹¹, mit R⁸ bis R¹¹ unabhängig voneinander stehend für einen C₁₋₄-Kohlenwasserstoffrest, steht,
- sulfatierte Fettsäurealkylenglykolester der Formel R¹²CO(AlkO)ₙSO₃M, in der R¹²CO-für einen linearen oder verzweigten, aliphatischen, gesättigten und/oder ungesättigten C₆₋₂₂-Acylrest, Alk für CH₂CH₂, CHCH₃CH₂ und/oder CH₂CHCH₃, n für Zahlen von 0,5 bis 5 und M für ein Kation steht, wie sie in der DE-OS 197 36 906 beschrieben sind,
- Monoglyceridsulfate und Monoglyceridethersulfate der Formel (II), wie sie z. B. in der EP-B1 0 561 825, der EP-B1 0 561 999, der DE-A1 42 04 700 oder von A. K. Biswas et al. in J.Am.Oil.Chem.Soc. 37, 171 (1960) und F. U. Ahmed in J.Am.Oil.Chem.Soc. 67, 8 (1990) beschrieben worden sind, 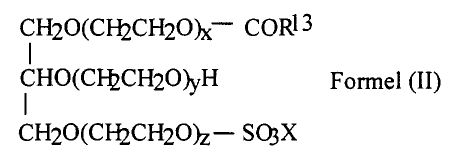 und in der R¹³CO für einen linearen oder verzweigten C₆₋₂₂-Acylrest, x, y und z in Summe für 0 oder für Zahlen von 1 bis 30, vorzugsweise 2 bis 10, und X für ein Alkali- oder Erdalkalimetall steht. Beispiele für erfindungsgemäß geeignete Monoglycerid(ether)sulfate sind die Umsetzungsprodukte von Laurinsäuremonoglycerid, Kokosfettsäuremonoglycerid, Palmitinsäuremonoglycerid, Stearinsäuremonoglycerid, Ölsäuremonoglycerid und Talgfettsäuremonoglycerid sowie deren Ethylenoxidaddukte mit Schwefeltrioxid oder Chlorsulfonsäure in Form ihrer Natriumsalze. Vorzugsweise werden Monoglyceridsulfate der Formel (II) eingesetzt, in der R⁸CO für einen linearen C₈₋₁₈-Acylrest steht.

Bevorzugte anionische Tenside sind die physiologisch verträglichen Salze, insbesondere die Na- und K-Salze linearer und verzweigter Fettsäuren mit 8 bis 30 C-Atomen (Seifen).

Für eine spezielle Ausführungsform der Erfindung als Kompaktreinigungsstift zur Reinigung des Gesichts, der Hände und des Körpers werden als oberflächenaktive Substanzen Tenside mit Reinigungswirkung ausgewählt. Die Zusammensetzung der Komponente (D) wird so gewählt, dass die reinigenden Tenside einen Anteil von 1 - 5 Gew.-% an der gesamten Stiftzusammensetzung haben. Der Stift wird auf die angefeuchtete Haut appliziert.

Der Anteil der teilchenförmigen Komponenten (E) an der Gesamtzusammensetzung beträgt 1 - 50 Gew.-%, bevorzugt 10 - 40 Gew.-% und besonders bevorzugt 15 - 40 Gew.-%, jeweils bezogen auf die gesamte Zusammensetzung.

Die enthaltenen teilchenförmigen Komponenten werden bevorzugt ausgewählt aus inerten, feinteiligen anorganischen und organischen Adsorbentien, Abrasivkomponenten und Pigmenten, sowie aus Mischungen der genannten Substanzen.

Teilchenförmige, zur Sebumadsorption geeignete anorganische und organische kosmetische Adsorbentien mit mittleren Partikeldurchmessern von 1 - 100 µm werden als mattierende Wirkstoffe zur nichttherapeutischen Behandlung fettiger Haut eingesetzt. Die Adsorbentien sind ausgewählt aus Kieselsäuren, z. B.Aerosil® -Typen, Kieselgelen, Siliciumdioxid, Tonen, z. B. Bentonite oder Kaolin, Magnesiumaluminiumsilikaten, z. B. Talkum, und Bornitrid, gegebenenfalls modifizierten Stärken und Stärkederivaten, Cellulosepulvern, Lactoglobulinderivaten, Polymerpulvern aus Polyolefinen, Polycarbonaten, Polyurethanen, Polyamiden, Polyestern, Polystyrolen, Polyacrylaten, (Meth)acrylat- oder (Meth)acrylat-Vinyliden-Copolymeren, die vernetzt sein können, Teflon oder Siliconen, sowie Mischungen der genannten Substanzen.

Polymerpulver auf Basis eines Polymethacrylat-Copolymers sind z. B. als Handelsprodukt Polytrap® 6603 (Dow Corning) erhältlich. Andere Polymerpulver, z. B. auf Basis von Polyamiden, sind unter der Bezeichnung Orgasol® 1002 (Polyamid-6) und Orgasol® 2002 (Polyamid-12) von Elf Atochem erhältlich. Weitere Polymerpulver, die sich für den erfindungsgemäßen Zweck eignen, sind z. B. Polymethacrylate (Micropearl® M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL® EP 400 von AKZO) oder auch Siliconpolymere (Silicone Powder X2-1605 von Dow Corning).

Bevorzugt eingesetzt werden Talkum, Siliciumdioxid, Kieselgel, Natrium-C₈₋₁₆-Isoalkylsuccinyllactoglobulinsulfonat, von Brooks Industries erhältlich als Handelsprodukt Biopol® OE, modifizierte Stärkederivate vom Typ DRY FLO® der National Starch and Chemical Company, Orgasol® und Polytrap® 6603. Besonders bevorzugt sind Talkum, Siliciumdioxid, Biopol® OE, Stärkederivate vom Typ DRY FLO® und das Polymerpulver Polytrap® 6603.

Weiterhin können die erfindungsgemäßen Zusammensetzungen sowohl farbige als auch farblose Pigmente enthalten. Einige der im folgenden genannten Pigmente dienen auch als UV-Absorber. Besonders bevorzugte farbige Pigmente sind ausgewählt aus den Eisenoxiden mit den Colour Index-Nummern Cl 77491 (Eisenoxid rot), Cl 77492 (Eisenoxidhydrat gelb) und Cl 77499 (Eisenoxid schwarz), aus Cl 77891 (Titandioxid) und Ruß. Andere bevorzugte Farbpigmente sind ausgewählt aus Cl 15510, Cl 15585, Cl 15850, Cl 15985, Cl 45170, Cl 45370, Cl 45380, Cl 45425, Cl 45430, Cl 73360, und Cl 75470. Die bevorzugten Pigmente mit UV-Absorbereigenschaften sind ausgewählt aus den Oxiden von Titan, Zink, Cer und Zirkon.

Die bevorzugten anorganischen Partikelsubstanzen sind hydrophil oder amphiphil. Vorteilhafterweise können sie oberflächlich beschichtet, insbesondere oberflächlich wasserabweisend behandelt ("gecoatet") sein. Beispiele hierfür sind mit Aluminiumstearat beschichtete Titandioxid-Pigmente (Handelsprodukt MT 100 T von der Firma Tayca), mit Dimethylpolysiloxan (Dimethicone) beschichtetes Zinkoxid, mit Dimethicone beschichtetes Bornitrid (Très BN® UHP 1106 von Carborundum), mit einem Gemisch aus Dimethylpolysiloxan und Silicagel (Simethicone) und Aluminiumoxidhydrat (Alumina) beschichtetes Titandioxid (Eusolex® T 2000 von Merck), mit Octylsilanol beschichtetes Titandioxid oder sphärische Polyalkylsesquioxan-Partikel (Aerosil® R972 und Aerosil® 200V von Degussa).

Eine andere bevorzugte Ausführungsform der erfindungsgemäßen Stiftmasse eignet sich zur kosmetischen Behandlung und zur Verringerung von Hautproblemen wie Cellulite und ist besonders bevorzugt als Massagestift ausgebildet. Hierzu liegt der Anteil der Pigment- und absorbierenden Pudersubstanzen an der gesamten Stiftmasse bei 10 - 20 Gew.-%. Den Massageeffekt erzielen zum kosmetischen Hautpeeling geeignete partikelförmige Substanzen, die in einem Anteil von 2 - 15 Gew.-% an der gesamten Stiftmasse enthalten sind.

Die zum kosmetischen Hautpeeling geeigneten Substanzen werden erfindungsgemäß ausgewählt aus gemahlenen Pflanzenteilen wie Mandelkleie oder Weizenkleie, kristalliner Cellulose, gehärtetem Jojobaöl (Jojobabeads), Polymerkügelchen, bevorzugt aus Polyethylen oder Polyamid-11, mit mittleren Durchmessern von 90 - 600 µm und aus wirkstoffhaltigen Mikro- oder Millikapseln, die petrochemische Polymere (z. B. aus Polyamid wie Nylon-11) und/oder Biopolymere wie Gelatine, Pektin, pflanzlichen Gummen, Alginaten und Carrageenan enthalten. Bevorzugt als Peelingsubstanzen eingesetzt werden Mandelkleie, Weizenkleie, gehärtetes Jojobaöl und Polymerkügelchen, insbesondere Polyethylenkügelchen. Ebenfalls bevorzugt sind wirkstoffhaltige Mikro- oder Millikapseln. Die handelsüblichen Kapseln liegen häufig als wässrige Polymer-Dispersion vor, beispielweise die besonders bevorzugten Millicapsules® der Firma Lipotec SA (INCl-Bezeichnung: Aqua, Tocopheryl Acetate, Glycerine, Carbomer, Sebacic Acid, Agar, Green Colourant, Alginic Acid).

In bevorzugten Ausführungsformen der erfindungsgemäßen Stifte sind bis zu 5 Gew.-% Wasser, bezogen auf die Gesamtzusammensetzung, enthalten. In einer weiteren bevorzugten Ausführungsform der erfindungsgemäßen Stiftmasse sind bis zu 15 Gew.-% kosmetischer oder dermatologischer Wirkstoffe, bezogen auf die Gesamtzusammensetzung, enthalten.

Die kosmetischen oder dermatologischen Wirkstoffe weisen besonders bevorzugt eine pflegende, adstringierende, sebumregulierende oder durchblutungsfördernde Wirkung auf.

Die Wirkstoffe sind ausgewählt aus Vitaminen, Pro-Vitaminen und Vitaminvorstufen, wasser- und öllöslichen Pflanzenextrakten, Mono-, Di- Oligo- und Polysacchariden und deren Derivaten, aus Proteinhydrolysaten und weiteren Komponenten mit kosmetischer und dermatologischer Wirkung.

Von den Vitaminen, Pro-Vitaminen und Vitaminvorstufen sind erfindungsgemäß solche bevorzugt, die üblicherweise den Gruppen A, B, C, E, F und H zugeordnet werden.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester wie das Palmitat und das Acetat in Betracht.

Zur Vitamin B-Gruppe oder Vitamin B-Komplex gehören u. a.
- Vitamin B₁ (Thiamin)
- Vitamin B₂ (Riboflavin)
- Vitamin B₃.

Unter der Bezeichnung Vitamin B₃ werden häufig die Verbindungen Nicotinsäure und Nicotinsäureamid (Niacinamid) geführt. In den erfindungsgemäßen Mitteln werden neben diesen beiden Substanzen bevorzugt die folgenden Derivate eingesetzt: Nicotinylalkohol, Nicotinsäureester, Nicotinylaminosäuren, Nicotinylalkoholester von Carbonsäuren, Nicotinsäure-N-oxid und Niacinamid-N-oxid. Die Nicotinsäureester tragen bevorzugt einen C₁₋₂₂-Alkanolrest und besonders bevorzugt einen C₁₋₆-Alkanolrest, wobei der Alkanolreste geradkettig oder verzweigt, cyclisch oder acyclisch, gesättigt, ungesättigt oder aromatisch, nichtsubstituiert oder substituiert sein kann. Einige der Ester der Nicotinsäure haben eine gefäßerweiternde Wirkung, die in bestimmten Präparaten zur nichttherapeutischen kosmetischen Hautbehandlung genutzt wird. Bevorzugte gefäßerweitemde Nicotinsäureester sind Tocopherolnicotinat und Inositolhexanicotinat, von denen das Tocopherolnicotinat erfindungsgemäß besonders bevorzugt ist.

Weitere Niacinamid-Derivate resultieren aus der Substition der Wasserstoffatome der Amidgruppe, z. B. die Nicotinylhydroxamsäure. Bevorzugte Nicotinylalkoholester sind die Salicylate, Acetate, Glykolate und Palmitate.
- Vitamin B₅ (Pantothensäure und Panthenol). Im Rahmen dieser Gruppe wird bevorzugt das Panthenol eingesetzt. Erfindungsgemäß einsetzbare Derivate des Panthenols sind insbesondere die Ester und Ether des Panthenols sowie kationisch derivatisierte Panthenole. Besonders bevorzugte Vertreter sind beispielsweise das Panthenoltriacetat, der Panthenolmonoethylether und dessen Monoacetat sowie die in der WO 92/13829 offenbarten kationischen Panthenolderivate. Auch Pantolacton, eine Vorstufe der Pantothensäure, kann bevorzugt eingesetzt werden.
- Vitamin B₆ (Pyridoxin sowie Pyridoxamin und Pyridoxal).

Vitamin C (Ascorbinsäure). Ascorbinsäure zur kosmetischen Anwendung wird bevorzugt in Form des Palmitinsäureesters eingesetzt.

Vitamin E (Tocopherole, insbesondere α-Tocopherol). In den erfindungsgemäßen Mitteln werden bevorzugt Tocopherol und seine Derivate, insbesondere Ester wie das Acetat, das Nicotinat, das Phosphat und das Succinat, eingesetzt.

Vitamin F. Unter dem Begriff "Vitamin F" werden üblicherweise essentielle Fettsäuren, insbesondere Linolsäure, Linolensäure und Arachidonsäure, verstanden.

Vitamin H. Als Vitamin H wird die Verbindung (3a*S*,4*S*, 6a*R*)-2-Oxohexahydrothienol[3,4-*d*]-imidazol-4-valeriansäure bezeichnet, für die sich aber zwischenzeitlich der Trivialname Biotin durchgesetzt hat. Biotin ist in den erfindungsgemäßen Mitteln bevorzugt in Mengen von 0,0001 bis 0,1 Gew.-%, insbesondere in Mengen von 0,001 bis 0,01 Gew.-% enthalten.

Bevorzugt enthalten die erfindungsgemäßen Mittel Vitamine, Provitamine und Vitaminvorstufen aus den Gruppen A, B, E und H. Panthenol und seine Derivate sowie Nicotinsäureamid und Biotin sind besonders bevorzugte Vitaminwirkstoffe.

Pflanzenextrakte werden üblicherweise durch Extraktion der gesamten Pflanze, in einzelnen Fällen aber auch ausschließlich aus Blüten und/oder Blättern und/oder anderen Pflanzenteilen, hergestellt. Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflege- und Waschmittel e.V. (IKW), Frankfurt, beginnenden Tabelle aufgeführt sind. Erfindungsgemäß sind vor allem die Extrakte aus dem Meristem, also dem teilungsfähigen Bildungsgewebe der Pflanzen, und speziellen Pflanzen wie Hamamelis, Kamille, Ringelblume, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Mango, Aprikose, Limone, Weizen, Melone, Orange, Grapefruit, Avocado, Rosmarin, Birke, Buchensprossen, Malve, Wiesenschaumkraut, Schafgarbe, Quendel, Thymian, Melisse, Hauhechel, Eibisch, Blättern der Schwarzen Johannisbeere, Huflattich, Fünffingerkraut, Ginseng, Ingwerwurzel und grünem Tee bevorzugt. Vorteilhaft eingesetzt werden können auch die Extrakte aus Blaualgen (Cyanobakterien), z. B. Spirulina, Rotalgen und Grünalgen verwendet werden. Die Algen können dabei sowohl natürlichen Ursprungs als auch biotechnologisch produziert und ggf. modifiziert sein. Besonders bevorzugt sind die Extrakte aus Spirulina, Hamamelis, Meristem, Kamille, Ringelblume, Paeonie, Aloe Vera, Roßkastanie, Salbei, Zimtbaum (cinnamon tree), grünem Tee, Weidenrinde und Chrysanthemen. Die erfindungsgemässen Mittel können auch Mischungen aus mehreren, insbesondere aus zwei, verschiedenen Pflanzenextrakten enthalten. Als Extraktionsmittel zur Herstellung der genannten Pflanzenextrakte können u. a. Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol, Propylenglykol und Butylenglykol und zwar sowohl als alleiniges Extraktionsmittel als auch in Mischung mit Wasser, bevorzugt. DieWasserdampfdestillation fällt erfindungsgemäß unter die bevorzugen Extraktionsverfahren. Die Pflanzenextrakte können erfindungsgemäß sowohl in reiner als auch in verdünnter Form eingesetzt werden; besonders bevorzugt wird der Kamillenwirkstoff Bisabolol als Reinsubstanz eingesetzt.

Besonders bevorzugt eingesetzte Mono-, Di-, Oligosaccharide und Polysaccharide und ihre Derivate sind Glucose, Galactose, Fructose, Fucose und Lactose, Honig, Honigextrakte und fucosereiche Polysaccharide.

Die erfindungsgemäß bevorzugten Proteinhydrolysate können tierischer Herkunft sein, beispielsweise aus Collagen, Fischcollagen, Milch oder Keratin, aus Pflanzen, beispielsweise aus Weizen, Mais, Reis, Kartoffeln, Soja, Mandeln oder Algen, oder aus Hefen und durch biotechnologische Prozesse gewonnen werden. Besonders bevorzugt sind pflanzliche, Hefe- und Chitinproteinhydrolysate. Die Proteinhydrolysate können auch kationisiert eingesetzt werden. Bevorzugt sind solche kationischen Proteinhydrolysate, deren zugrunde liegender Proteinanteil ein Molekulargewicht von 100 bis zu 25000 Dalton, bevorzugt 250 bis 5000 Dalton aufweist. Besonders bevorzugte kationische Proteinhydrolysate sind Chitosane, also teildeacetylierte Chitinderivate, z. B. als Handelsprodukt Hydagen® erhältlich.

Besonders bevorzugte Komponenten mit kosmetischer und dermatologischer Wirkung sind außerdem Allantoin, Pyrrolidoncarbonsäure (PCA) und ihre Ester mit Fettalkoholen sowie Isolaurylthioether und seine Derivate.

Sebumregulierende Wirkstoffe werden im Sinne der Erfindung besonders bevorzugtausgewählt aus wasser- und öllöslichen Extrakten aus Hamamelis, Klettenwurzel und Brennessel, Zimtbaumextract (z. B. Sepicontrol® A5 von der Firma Seppic), Chrysanthemenextrakt (z. B. Laricyl® von Laboratoires Sérobiologiques) und aus handelsüblichen Wirkstoffmischungen, z. B. Asebiol® BT 2 (INCI: Aqua, Hydrolyzed Yeast Protein, Pyridoxine, Niacinamide, Glycerin, Panthenol, Allantoin, Biotin) der Firma Laboratoires Sérobiologiques und Antifettfaktor® COS-218/2-A (von Cosmetochem, INCI: Aqua, Cetyl-PCA, PEG-8 Isolauryl Thioether, PCA, Cetyl Alcohol).

Adstringierende Wirkstoffe werden im Sinne der Erfindung besonders bevorzugt ausgewählt aus wasser- und öllöslichen Extrakten aus Hamamelis, Weidenrinde, Eichenrinde, Fünffingerkraut, Spitzwegerich und Salbei.

Antimikrobielle Wirkstoffe werden im Sinne der Erfindung besonders bevorzugt ausgewählt aus wasser- und öllöslichen Extrakten der Blätter der Schwarzen Johannisbeere, Kamillenblüten, Gewürznelken, Klettenwurzel, Stiefmütterchen, Spitzwegerich und grünem Tee sowie aus Terpenalkoholen, z. B. Farnesol, und Bestandteilen des Lindenblütenöls. Darüber hinaus lassen sich Organohalogenverbindungen sowie Organohalogenide, quartäre Ammoniumverbindungen und Zinkverbindungen zur Keimhemmung einsetzen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Cloflucarban, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Laurylpyridiniumchlorid, Laurylisoquinoliniumbromid, Cetylpyridiniumchlorid und Methylbenzedoniumchlorid. Desweiteren sind Phenol, Phenoxyethanol, Dinatriumdihydroxyethylsulfosuccinylundecylenat, Natriumbicarbonat, Zinklactat, Zinkphenolsulfonat und Natriumphenolsulfonat, Ketoglutarsäure, Chlorophyllin-Kupfer-Komplexe, Glycerinmonoalkylether, Fettsäuren sowie Carbonsäureester des Mono-, Di- und Triglycerins (z. B. Glycerinmonolaurat, Diglycerinmonocaprinat) einsetzbar.

Die durchblutungsfördernden Substanzen werden ausgewählt aus Nicotinsäurederivaten mit vasodilatorischer Wirkung, Capsaicin, Extrakten aus Chilischoten (red pepper), Rutin und Rutinderivaten, Coffein und Rosskastanienextrakt sowie Mischungen hiervon. Ein erfindungsgemäß besonders bevorzugtes durchblutungsförderndes Nicotinsäurederivat ist das Vitamin E-nicotinat (Tocopherolnicotinat). Aufgrund ihrer verschieden starken Wirkung werden diese Stoffe in unterschiedlichen Gewichtsanteilen eingesetzt. Die bevorzugten Einsatzmengen betragen für Vitamin E-nicotinat 0,1-2,0 Gew.-% Reinsubstanz und für das Chiliextraktpulver 0,01 - 0,1 Gew.-%, bezogen auf die gesamte Stiftzusammensetzung. Coffein, Rutin und Rosskastanienextrakt werden bevorzugt in Form wässrig-alkoholischer Lösungen mit einem Aktivsubstanzgehalt von 0,1 - 10 Gew.-% eingesetzt. Erfindungsgemäß beträgt der Anteil dieser handelsüblichen Lösungen an der Stiftmasse 1 - 10 Gew.-%, bezogen auf die gesamte Stiftzusammensetzung.

Die durchblutungsfördemden Substanzen können auch der besonderen Ausführungsform der Erfindung als Massagestift zusätzlich zu den Peelingkörpern beigefügt werden.

Eine weitere bevorzugte Ausführungsform des Stiftes ermöglicht das handliche und gut zu dosierende Applizieren von ätherischen Ölen oder Duftstoffen auf der Haut zur Erfrischung und Steigerung des Wohlbefindens, z. B. im Rahmen der Aromatherapie. Der Stiftbasis werden 0,5 - 7 Gew.-%, bevorzugterweise 1 - 5 Gew.-%, bezogen auf die gesamte Stiftzusammensetzung, an ätherischen Ölen und Duftstoffen zugesetzt, ausgewählt aus Menthol, Mentholderivaten wie Menthyllactat (z. B. als Handelsprodukt Frescolat® -ML erhältlich), Ysopöl, Pfefferminzöl, Thymol, Grapefruitöl, Mandarinenöl, Orangenöl, Zitronenöl, Bergamotteöl, Nelkenöl, Rosmarinöl, Zypressenöl, Zedernöl und Lavendelöl. Diese Aufzählung hat keinen limitierenden Charakter.

In einer anderen bevorzugten Ausführungsform enthält die Stiftgrundlage mindestens eine Substanz, die beim Vermischen mit Wasser Hydratationswärme entwickelt.

Entsprechende kosmetische Mittel in kompakter Form sind bislang noch nicht beschrieben. Der erfindungsgemäße Stift, der zusätzlich Hydratationswärme entfaltende Substanzen enthält und auf die feuchte Haut appliziert wird, bietet mehrere Vorteile. Durch die freigesetzte Wärme kommt es zu einem sensorisch angenehmen Hauteindruck. In Kombination mit den bereits beschriebenen erfindungsgemäßen Inhaltsstoffen wird z.B. eine Verbesserung des Reinigungseffektes, eine intensivere Freisetzung von Aroma- und Duftstoffen und eine verbesserte Wirkung enthaltener hautkosmetischer Aktivsubstanzen erzielt.

Die erfindungsgemäß einsetzbaren Wärme entwickelnden Substanzen sind ausgewählt aus wasserfreien, mit Wasser mischbaren Hydroxylverbindungen, Zeolithen und Salzen mit einer negativen Lösungsenthalpie.

Zu den geeigneten Hydroxylverbindungen zählen Glycole, Glycolether, Polyole mit 2-6 Kohlenstoffatomen und Polyalkylenglycole. Geeignete Glycole sind Ethylenglycol, Propandiole und Butandiole. Geeignete Glycolether sind z. B. Ethylglycol, Ethyldiglycol, Diethylenglycol, Triethylenglycol und Dipropylenglycol. Geeignete Polyole sind z. B. Glycerin, Erythrit, Pentaerythrit, Trimethylolpropan, Diglycerin und Sorbit. Geeignete Polyalkylenglycole sind z. B. die flüssigen Polyethylenglycole, beispielsweise PEG-600, ein Polyethylenglycol mit einem Molekulargewicht von 600 D und als Handelsprodukt Lutrol® 400 von der Firma BASF erhältlich, die Polypropylenglycole und die Anlagerungsprodukte von Ethylenoxid an Propylenglycol oder an Polypropylenglycole, jeweils mit Molekulargewichten bis ca. 1000 D. Um hinsichtlich der Wärmeentwicklung ein optimales Anwendungsprofil zu erhalten, werden bevorzugt Mischungen von zwei oder mehr unterschiedlichen Hydroxylverbindungen eingesetzt. Gemische von Propylenglycol-1,2, Butylenglycol-1,3, Polyethylenglycol und Ethoxydiglycol sind besonders bevorzugt.

Eine erfindungsgemäß besonders bevorzugte Zeolith-Verbindung ist aktivierter Zeolith A.

Weiterhin sind erfindungsgemäß ganz oder teilweise dehydratisierte Salze geeignet, die in Wasser Hydrate bilden. Solche Salze sind z. B. Ortho- und Pyrophosphate, Carbonate und Sesquicarbonat, Borate, Chloride und Sulfate von Alkalimetallen, z. B. des Natriums. Geeignete Salze sind auch Alkalimetallcitrate und -acetate. Weitere geeignete Salze sind Zinkcitrat, Zinksulfat, Zinknitrat, Calciumchlorid, Calciumsulfat, Magnesiumchlorid, Magnesiumsulfat und Aluminiumsulfat. Im Sinne der Erfindung ist als hydratisierendes Salz Natrium-, Magnesium- oder Aluminiumsulfat oder ein Gemisch davon bevorzugt.

In einer besonders bevorzugten Ausführungsform wird ein Gemisch aus geeigneten Hydroxylverbindungen und hydratisierenden Salzen im Mengenverhältnis 4:1 bis 6:1, besonders bevorzugt 5:1 eingesetzt. Das gesamte Wärme entwickelnde Gemisch liegt in Anteilen von bevorzugt 1 - 15 Gew.-%, besonders bevorzugt 7 - 10 Gew.-%, bezogen auf die gesamte Stiftzusammensetzung, vor.

In einer bevorzugten Ausführungsform enthält die Stiftmasse 0,5 bis 10 Gew.-%, besonders bevorzugt 1 - 5 Gew.-%, jeweils bezogen auf die Gesamtzusammensetzung, an weiteren kosmetischen Ölkomponenten.

Als zusätzliche Ölkomponenten geeignet sind lineare oder verzweigte primäre Alkohole, C₁₂₋₂₄-Alkanole, Guerbetalkohole auf Basis von Fettalkoholen mit 6 bis 18, vorzugsweise 8 bis 10 Kohlenstoffatomen, C₁₂₋₂₄-Alkandiole, Ester von linearen C₆-C₂₀-Fettsäuren mit linearen C₆-C₂₀-Fettalkoholen, Ester von verzweigten C₆-C₁₃-Carbonsäuren mit linearen C₆-C₂₀-Fettalkoholen, Ester von linearen C₆-C₁₈-Fettsäuren mit verzweigten Alkoholen, insbesondere 2-Ethylhexanol, Ester von linearen und/oder verzweigten Fettsäuren mit mehrwertigen Alkoholen (wie z. B. Dimerdiol oder Trimertriol) und/oder Guerbetalkoholen, Hydroxycarbonsäurealkylester (wobei die Vollester der Glycolsäure, Milchsäure, Äpfelsäure, Weinsäure oder Citronensäure bevorzugt sind, aber auch Ester der β-Hydroxypropionsäure, der Tartronsäure, der D-Gluconsäure, Zuckersäure, Schleimsäure oder Glucuronsäure geeignet sind und besonders bevorzugt die Ester von C₁₂-C₁₅-Fettalkoholen, z. B. als Handelsprodukt Cosmacol® der EniChem, Augusta Industriale, sind), weiterhin C₁₂₋₂₄-Etheralkohole oder C₁₂₋₂₄-Dialkylether, pflanzliche Öle, Triglyceride auf Basis C₆-C₁₀-Fettsäuren, Guerbetcarbonate, substituierte Cyclohexane, und/oder aliphatische bzw. naphthenische Kohlenwasserstofföle wie Polydecen (z.B. Nexbase® 2004 FG der Firma Fortum).

Weiterhin können die Stiftmassen Antioxidantien enthalten, die sowohl zur Stabilisierung der Fette gegen Autoxidation als auch als Radikalfänger zur vorbeugenden Hautpflege dienen. Besonders vorteilhaft werden die Antioxidantien gewählt aus der Gruppe, bestehend aus Aminosäuren (z. B. Glycin, Histidin, Tyrosin, Tryptophan) und deren Derivate, Imidazol und Imidazolderivate (z. B. Urocaninsäure), Peptide wie z. B. D,L-Carnosin, D-Carnosin, L-Camosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und weitere Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis µmol/kg), ferner Metallchelatoren (z. B. α-Hydroxyfettsäuren EDTA, EGTA, Phytinsäure, Lactoferrin), α-Hydroxysäuren (z. B. Zitronensäure, Milchsäure, Äpfelsäure), Huminsäuren, Gallensäure, Gallenextrakte, Gallussäureester (z. B. Propyl-, Octyl- und Dodecylgallat), Flavonoide, Catechine, Bilirubin, Biliverdin, und deren Derivate, ungesättigte Fettsäuren und deren Derivate (z. B. γ-Linolensäure, Linolsäure, Arachidonsäure, Ölsäure), Folsäure und deren Derivate, Hydrochinon und dessen Derivate (z. B. Arbutin), Ubichinon und Ubichinol sowie deren Derivate, Vitamin C und dessen Derivate (z. B. Ascorbylpalmitat,-stearat, -dipalmitat, -acetat, Mg-Ascorbylphosphate, Natrium- und Magnesiumascorbat, Dinatriumascorbylphosphat und -sulfat, Kaliumascorbyltocopherylphosphat, Chitosanascorbat), Isoascorbinsäure und deren Derivate, Tocopherole und deren Derivate (z. B. Tocopherylacetat, -linoleat, -oleat und -succinat, Tocophereth-5, Tocophereth-10, Tocophereth-12, Tocophereth-18, Tocophereth-50, Tocophersolan), Vitamin A und Derivate (z. B. Vitamin-A-Palmitat), das Coniferylbenzoat des Benzoeharzes, Rutin, Rutinsäure und deren Derivate, Dinatriumrutinyldisulfat, Zimtsäure und deren Derivate (z. B. Ferulasäure, Ethylferulat, Kaffeesäure), Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und Zink-Derivate (z. B. ZnO, ZnSO₄), Selen und Selen-Derivate (z. B. Selenmethionin), Stilbene und Stilben-Derivate (z. B. Stilbenoxid, trans-Stilbenoxid).

Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden.

Die Gesamtmenge der Antioxidantien in den erfindungsgemäßen Zusammensetzungen beträgt 0,001 - 10 Gew.-%, vorzugsweise 0,05 - 5 Gew.-% und insbesondere 0,1 - 2 Gew.-%, bezogen auf das Gesamtgewicht der Zubereitung.

Die nachfolgenden Beispiele sollen den Gegenstand der Erfindung näher erläutern, ohne ihn hierauf zu beschränken.

| **Liste der verwendeten Bestandteile** | | |
|---|---|---|
| Bestandteil | INCI-Bezeichnung | Hersteller/Lieferant |
| Dow Corning 245 Fluid | Cyclopentasiloxan | Dow Corning |
| Cutina® HR | Hydrogenated Castor Oil | Cognis Deutschland GmbH |
| Novata® AB Pastillen | Cocoglycerides | Cognis Deutschland GmbH |
| Cutina® E 24 PF | PEG-20 Glyceryl Stearate | Cognis Deutschland GmbH |
| Talkum Pharma G | Talc | China National Metals & |
| | (Magnesiumsilikathydrat) | Minerals |
| Dry Flo® Plus | Aluminium Starch | National Starch & Chemical |
| | Octenylsuccinate | Company |
| Biopol® OE | Natrium-C₈₋₁₆-Isoalkylsuccinyl | Brooks Industries |
| | lactoglobulinsulfonat | |
| Herbasol® Destillat Kamille | Aqua, Alcohol denat., | Cosmetochem |
| | Chamomilla recutita | |
| Herbasol® Extrakt Pfefferminz | Isopropyl myristate, | Cosmetochem |
| (öllöslich) | Mentha piperita | |
| Herbasol® Extrakt Hamamelis | Aqua, Propylene Glycol, | Cosmetochem |
| | Hamamelis virginiana | |
| Herbasol® Extrakt Horse | Aqua, Propylene Glycol, | Cosmetochem |
| Chestnut (Rosskastanien- | Aesculus Hippocastanum, | Cosmetochem |
| extrakt) | Phenoxyethanol, Methylparaben | |
| Asebiol® LS 2539 BT 2 | Aqua, Hydrolyzed Yeast | Laboratoires |
| | Protein, Pyridoxine, Niacin- | Sérobiologiques |
| | amide, Glycerin, Panthenol | |
| | Propylene Glycol, Allantoin, | |
| | Biotin. | |
| Sepicontrol® A5 | Zimtbaumextrakt | Seppic |
| | INCI: Capryloyl Glycine, Sarcosine, | |
| | Cinnamomum Zeylanicum | |

### Beispiele

Alle Mengenangaben der Ausgangsprodukte beziehen sich auf die Substanzen in ihrer Handelsform (telle quelle).

| | Nr. 1 Mattierender und hautberuhigender Kosmetik-Stift [Gewichtsteile] | Nr. 2 Mattierender und sebumregulierender Kosmetik-Stift [Gewichtsteile] | Nr. 3 Mattierender und adstringierender Kosmetik-Stift [Gewichtsteile] |
|---|---|---|---|
| Dow Corning 245 Fluid | 17,4 | 22,0 | 23,0 |
| Cutina® HR | 6,0 | 6,0 | 6,0 |
| Novata® AB Pastillen | 17,0 | 11,0 | 13,0 |
| Cutina® E 24 PF | 2,0 | 2,0 | 2,0 |
| Ethanol, 96 %ig | 20,5 | 20,8 | 22,0 |
| Talkum Pharma G | 35,0 | 25,0 | 20,0 |
| Dry Flo® Plus | - | 10,0 | - |
| Biopol® OE | - | - | 10,0 |
| Herbasol® Destillat Kamille | 0,9 | - | - |
| Herbasol® Extrakt Pfefferminz (öllöslich) | 1,0 | - | 0,9 |
| d, I-alpha-Bisabolol | 0,1 | 0,1 | - |
| Herbasol® Extrakt Hamamelis | - | - | 1,0 1,0 |
| Weidenrindenextrakt | - | - | 2,0 |
| Asebiol® LS 2539 BT 2 | - | 2,0 | - |
| Sepicontrol® A5 | - | 1,0 | - |
| Parfum | 0,1 | 0,1 | 0,1 |

| | **Nr. 4** Massagestift mit Anti-Cellulite-Wirkung [Gewichtsteile] | **Nr. 5** Massagestift mit Anti-Cellulite-Wirkung [Gewichtsteile] | **Nr. 6** Wärme entwickelnder Stift [Gewichtsteile] |
|---|---|---|---|
| Dow Corning 245 Fluid | 33,0 | 22,0 | 24,0 |
| Cutina® HR | 10,0 | 12,0 | 11,6 |
| Novata® AB Pastillen | 18,0 | 23,0 | 17,5 |
| Cutina® E 24 PF | 4,0 | 4,0 | 5,9 |
| Ethanol, 96 %ig | 12,5 | 13,0 | 10,0 |
| Talkum Pharma G | 15,0 | 10,0 | 20,0 |
| ROVI-beads Jojoba 40/60 (Peeling-Partikel, ROVI GmbH) | 7,3 | 10,0 | - |
| Chiliextrakt-Pulver | 0,1 | - | - |
| Vitamin E-nicotinat | - | 0,9 | - |
| Herbasol® Extrakt Horse Chestnut | - | 5,0 | - |
| Glycerin, wasserfrei | - | - | 10,0 |
| Herbasol® Extrakt Pfefferminz (öllöslich) | - | - | 0,9 |
| Parfum | 0,1 | 0,1 | 0,1 |

| | **Nr. 7** Erfrischungs-Stift [Gewichtsteile] | **Nr. 8** Aromapflege-Stift [Gewichtsteile] | **Nr. 9** Kompaktreinigungs-Stift [Gewichtsteile] |
|---|---|---|---|
| Dow Corning 245 Fluid | 16 | 15 | 25,0 |
| Cutina® HR | 6,5 | 6,5 | 7,3 |
| Novata® AB Pastillen | 24,0 | 27,09 | 28,0 |
| Cutina® E 24 PF | 3,5 | 2,5 | 6,7 |
| Ethanol, 96 %ig | 27,0 | 27,0 | 12,0 |
| Talkum Pharma G | 10,0 | 10,0 | 15,0 |
| Dry Flo® Plus | 10,0 | 10,0 | - |
| Frescolat® * | 3,0 | - | - |
| Glucamate® DOE 120 ** | - | - | 4,0 |
| Orangenschalenöl *** | - | 2,0 | 2,0 |

| | | | |
|---|---|---|---|
| * Frescolat® Menthyllactat Haarmann & Reimer | | | |
| ** Glucamate® DOE 120 PEG-120 Methyl Glucose Amerchol Dioleate | | | |
| *** Orangenschalenöl Dragoco | | | |

### Herstellung der Stifte

Die Fett- und Wachskomponenten werden zusammen mit dem Siliconöl aufgeschmolzen und auf ca. 50 °C abgekühlt. Die noch flüssige Siliconöl-/Fettmischung wird mit dem Alkohol und den jeweiligen Duft-, Aroma- und Wirkstoffen homogen vermischt. Die Pudersubstanzen und gegebenenfalls Pigmente oder Peeling-Körper werden trocken vermengt und anschließend in die Stiftschmelze eingerührt. Die Mischung wird warm (ca. 50 °C) in die Stifthülsen gegossen und erstarrt dort zu einer festen Masse.

## Patentansprüche

1. Bei 40 °C formstabile, auf der Haut verstreichbare kosmetische Stiftmasse, enthaltend
(A) ein- oder mehrwertige C₂₋₄-Alkohole,
(B) im Bereich von 28 - 130 °C schmelzende Fettkomponenten,
(C) Siliconöle mit einem Siedepunkt unter 250 °C,
(D) oberflächenaktive Komponenten und
(E) teilchenförmige Komponenten.

2. Kosmetische Stiftmasse nach Anspruch 1, **dadurch gekennzeichnet, dass** weiterhin bis zu 15 Gew.-% kosmetischer oder dermatologischer Wirkstoffe, bezogen auf die Gesamtzusammensetzung, enthalten sind.

3. Kosmetische Stiftmasse nach Anspruch 1 oder 2, **dadurch gekennzeichnet, dass** bis zu 5 Gew.-% Wasser, bezogen auf die Gesamtzusammensetzung, enthalten ist.

4. Kosmetische Stiftmasse nach einem der Ansprüche 1 - 3, **dadurch gekennzeichnet, dass** die Alkoholkomponente (A) Ethanol ist.

5. Kosmetische Stiftmasse nach einem der Ansprüche 1 - 4, **dadurch gekennzeichnet, dass** die Alkoholkomponente (A) zu 10 - 30 Gew.-%, bezogen auf die Gesamtzusammensetzung, enthalten ist.

6. Kosmetische Stiftmasse nach einem der Ansprüche 1 - 5, **dadurch gekennzeichnet, dass** die Fettkomponente (B) mindestens einen unterhalb von 50 °C schmelzenden Fettstoff und mindestens einen im Bereich von 50 °C und höher schmelzenden Fettstoff umfasst.

7. Kosmetische Stiftmasse nach Anspruch 6, **dadurch gekennzeichnet, dass** die unterhalb von 50 °C schmelzenden Fettstoffe ausgewählt sind aus den Mono- Di-und Triglyceriden der C₁₂₋₁₄-Fettsäuren, aus Estern von gegebenenfalls hydroxylierten C₂₋₄-Carbonsäuren mit Lanolinalkoholen und C₁₂₋₁₈-Fettalkoholen, acetyliertem Lanolin, Estern von gesättigten und ungesättigten C₁₂₋₁₈-Fettsäuren mit gesättigten und ungesättigten C₁₂₋₁₈-Fettalkoholen, Estem von C₂₋₁₀-Fettsäuren mit C₁₄-Fettalkohol, C₁₄-Fettsäureestern, Cholesterol- oder Lanosterolestern von C₁₀₋₃₀-Fettsäuren und ethoxylierten C₁₂₋₂₀-Fettsäureglykolestern.

8. Kosmetische Stiftmasse nach Anspruch 6 oder 7, **dadurch gekennzeichnet, dass** die ab 50 °C schmelzenden Fettstoffe ausgewählt sind aus denTriglyceriden gesättigter und gegebenenfalls hydroxylierter C₁₆₋₃₀-Fettsäuren, aus Bienenwachs, synthetischem Bienenwachs, Candelillawachs, Carnaubawachs, Ozokerit, Mikrowachs, Ceresin und Paraffin sowie Mischungen hiervon.

9. Kosmetische Stiftmasse nach einem der Ansprüche 6 - 8, **dadurch gekennzeichnet, dass** das Mengenverhältnis von unterhalb von 50 °C schmelzenden zu ab 50 °C schmelzenden Fettstoffen 1,5:1 bis 4:1 beträgt.

10. Kosmetische Stiftmasse nach einem der Ansprüche 1 - 9, **dadurch gekennzeichnet, dass** die Fettkomponenten (B) zu 10 - 40 Gew.-%, bezogen auf die gesamte Zusammensetzung, enthalten sind.

11. Kosmetische Stiftmasse nach Anspruch 1, **dadurch gekennzeichnet, dass** die Siliconöle (C) mit einem Siedepunkt unter 250 °C zu 10 - 40 Gew.-%, bezogen auf die gesamte Zusammensetzung, enthalten sind.

12. Kosmetische Stiftmasse nach Anspruch 1, **dadurch gekennzeichnet, dass** die oberflächenaktiven Komponenten (D) zu 1 - 10 Gew.-%, bezogen auf die gesamte Zusammensetzung, enthalten sind.

13. Kosmetische Stiftmasse nach Anspruch 1, **dadurch gekennzeichnet, dass** die oberflächenaktiven Komponenten (D) ausgewählt sind aus nichtionischen und anionischen oberflächenaktiven Komponenten sowie Mischungen hiervon.

14. Kosmetische Stiftmasse nach Anspruch 13, **dadurch gekennzeichnet, dass** die nichtionischen oberflächenaktiven Komponenten (D) ausgewählt sind aus C₁₂-C₃₀-Fettsäuremono- und -diestern von Anlagerungsprodukten von 1 bis 30 Mol Ethylenoxid an Glycerin, aus Polyglycerinen und Polyglycerinderivaten, Alkyl-(oligo)-glucosiden, Gemischen von Alkyl-(oligo)-glucosiden und Fettalkoholen, Partialestern von Zuckern, Zuckerderivaten und Zuckeralkoholen mit gesättigten C₈₋₂₂-Fettsäuren, sowie beliebigen Mischungen der genannten Substanzen.

15. Kosmetische Stiftmasse nach Anspruch 13, **dadurch gekennzeichnet, dass** die oberflächenaktiven anionischen Komponenten (D) ausgewählt sind aus den physiologisch verträglichen Salzen linearer und verzweigter C₈₋₃₀-Fettsäuren.

16. Kosmetische Stiftmasse nach einem der Ansprüche 1 - 15, **dadurch gekennzeichnet, dass** die teilchenförmigen Komponenten (E) zu 1 - 50 Gew.-%, bezogen auf die gesamte Zusammensetzung, enthalten sind.

17. Kosmetische Stiftmasse nach einem der Ansprüche 1 - 15, **dadurch gekennzeichnet, dass** die teilchenförmigen Komponenten (E) ausgewählt sind aus inerten, feinteiligen anorganischen und organischen Adsorbentien, Abrasivkomponenten und Pigmenten, sowie Mischungen der genannten Substanzen.

18. Kosmetische Stiftmasse nach Anspruch 17, **dadurch gekennzeichnet, dass** die anorganischen Adsorbentien und Abrasivkomponenten ausgewählt sind aus Kieselsäuren, Kieselgelen, Siliciumdioxid, Tonen, Magnesiumaluminiumsilikaten und Bornitrid sowie Mischungen der genannten Substanzen.

19. Kosmetische Stiftmasse nach Anspruch 17, **dadurch gekennzeichnet, dass** die organischen Adsorbentien und Abrasivkomponenten ausgewählt sind aus gegebenenfalls modifizierten Stärken und Stärkederivaten, kristalliner Cellulose, Cellulosepulvern, Lactoglobulinderivaten, gemahlenen Pflanzenteilen, gehärtetem Jojobaöl (Jojobabeads), Polymerpartikeln aus Polyolefinen, Polycarbonaten, Polyurethanen, Polyacrylaten, (Meth)acrylat- oder (Meth)acrylat-Vinyliden-Copolymeren, die vernetzt sein können, Polyestern, Polyamiden, Polystyrolen, Teflon oder Siliconen, und Mikro- oder Millikapseln, die petrochemische Polymere und/oder Biopolymere und gegebenenfalls kosmetische Wirkstoffe enthalten, sowie aus Mischungen der genannten Substanzen.

20. Kosmetische Stiftmasse nach Anspruch 17, **dadurch gekennzeichnet, dass** die Pigmente ausgewählt sind aus den Oxiden von Titan, Zink, Cer und Zirkon und den Eisenoxiden mit den Colour Indices Cl 77491, Cl 77492 und Cl 77499, aus Ruß und den Pigmenten mit den Colour Indices Cl 15510, Cl 15585, Cl 15850, Cl 15985, Cl 45170, Cl 45370, Cl 45380, Cl 45425, Cl 45430, Cl 73360, und Cl 75470, sowie aus Mischungen hiervon.

21. Kosmetische Stiftmasse nach einem der Ansprüche 2 - 20, **dadurch gekennzeichnet, dass** die kosmetischen und dermatologischen Wirkstoffe ausgewählt sind aus pflegenden, adstringierenden und sebumregulierenden Wirkstoffen.

22. Kosmetische Stiftmasse nach Anspruch 21, **dadurch gekennzeichnet, dass** die kosmetischen und dermatologischen Wirkstoffe ausgewählt sind aus Vitaminen und Vitaminvorstufen, wasser- und öllöslichen Pflanzenextrakten, Mono-, Di-, Oligo-und Polysacchariden und deren Derivaten, Proteinhydrolysaten und einer Gruppe von Komponenten, ausgewählt aus Allantoin, Pyrrolidoncarbonsäure (PCA) und ihren Estern mit Fettalkoholen, Isolaurylthioether und seinen Derivaten sowie aus Mischungen der genannten Substanzen.

23. Kosmetische Stiftmasse nach einem der Ansprüche 2 - 20, **dadurch gekennzeichnet, dass** die kosmetischen oder dermatologischen Wirkstoffe durchblutungsfördernde Substanzen sind, ausgewählt aus Nicotinsäurederivaten, Capsaicin, Extrakten aus Chilischoten (red pepper), Rutin und Rutinderivaten, Coffein und Rosskastanienextrakt, sowie Mischungen hiervon.

24. Kosmetische Stiftmasse nach einem der Ansprüche 1 - 23, **dadurch gekennzeichnet, dass** zusätzlich mindestens ein ätherisches Öl, ausgewählt aus Menthol und Mentholderivaten, Ysopöl, Pfefferminzöl, Thymol, Grapefruitöl, Mandarinenöl, Orangenöl, Zitronenöl, Bergamotteöl, Nelkenöl, Rosmarinöl, Zypressenöl, Zedernöl und Lavendelöl, in einer Menge von 0,5 - 7 Gew.-%, bezogen auf die Gesamtzusammensetzung, enthalten ist.

25. Kosmetische Stiftmasse nach einem der Ansprüche 1 - 24, **dadurch gekennzeichnet, dass** zusätzlich mindestens eine wasserfreie Komponente, die Hydratationswärme entwickelt, enthalten ist.

26. Kosmetische Stiftmasse nach Anspruch 25, **dadurch gekennzeichnet, dass** die Komponenten, die beim Vermischen mit Wasser Hydratationswärme erzeugen, ausgewählt sind aus wasserfreien Glycolen, Glycolethern, Polyolen mit 2 - 6 Kohlenstoffatomen, Polyalkylenglycolen, wasserfreien Zeolithen und wasserfreien Ortho- und Pyrophosphaten, Carbonaten, Sesquicarbonat, Boraten, Alkalimetallchloriden, -sulfaten, -citraten und -acetaten, Zinkcitrat, Zinksulfat, Zinknitrat, Calciumchlorid, Calciumsulfat, Magnesiumchlorid, Magnesiumsulfat und Aluminiumsulfat sowie Mischungen hiervon.

27. Kosmetische Stiftmasse nach Anspruch 25, **dadurch gekennzeichnet, dass** die Hydratationswärme erzeugenden Komponenten in einer Menge von 1 - 15 Gew.-%, bezogen auf die Gesamtzusammensetzung, enthalten sind.

28. Kosmetische Stiftmasse nach einem der Ansprüche 1 - 27, **dadurch gekennzeichnet, dass** weitere kosmetische Ölkomponenten in einer Menge von 0,5 bis 10 Gew.-%, bezogen auf die Gesamtzusammensetzung, enthalten sind.

29. Kosmetische Stiftmasse nach Anspruch 28, **dadurch gekennzeichnet, dass** die zusätzlichen Ölkomponenten ausgewählt sind aus linearen oder verzweigten primären Alkoholen, C₁₂₋₂₄-Alkanolen, Guerbetalkoholen auf Basis von C₆₋₁₈-Fettalkoholen, C₁₂₋₂₄-Alkandiolen, Estern von linearen C₆-C₂₀-Fettsäuren mit linearen C₆-C₂₀-Fettalkoholen, Estern von verzweigten C₆-C₁₃-Carbonsäuren mit linearen C₆-C₂₀-Fettalkoholen, Estern von linearen C₆-C₁₈-Fettsäuren mit verzweigten Alkoholen, Estern von linearen und/oder verzweigten Fettsäuren mit mehrwertigen Alkoholen und/oder Guerbetalkoholen, Hydroxycarbonsäurealkylestern, C₁₂₋₂₄-Etheralkoholen, C₁₂₋₂₄-Dialkylethern, pflanzlichen Ölen, Triglyceriden von C₆-C₁₀-Fettsäuren, Guerbetcarbonaten, substituierten Cyclohexanen, aliphatischen und naphthenischen Kohlenwasserstoffölen, sowie Mischungen hiervon.

30. Verwendung einer kosmetischen Zusammensetzung nach einem der Ansprüche 1 - 27 zur antibakteriellen Behandlung oder mattierenden kosmetischen Pflege fettiger, unreiner Haut.

31. Verwendung einer kosmetischen Zusammensetzung nach einem der Ansprüche 17 - 29 zur Steigerung der Hautdurchblutung, Minderung von Cellulite und Verbesserung des Hautzustandes.

32. Verwendung einer kosmetischen Zusammensetzung nach Anspruch 24 zur Erfrischung oder zur Steigerung des Wohlbefindens durch Aromen.
